# EUROPEAN PATENT APPLICATION

(11) **EP 4 660 936 A1**
(43) Date of publication of application: **10.12.2025**
(21) Application number: 23919918.5
(22) Date of filing: 06.12.2023
(51) Int. Cl.: G06T 7/00, C12M 1/34, C12Q 1/04, G01N 33/48, G06N 20/00

(54) **LEARNING SUPPORT DEVICE, METHOD FOR OPERATING LEARNING SUPPORT DEVICE, AND PROGRAM FOR OPERATING LEARNING SUPPORT DEVICE**

(30) Priority: 30.01.2023 JP 2023012250
(71) Applicant: FUJIFILM Corporation, Tokyo 106-8620 (JP)
(72) Inventor: INOUE, Tsutomu, Ashigarakami-gun, Kanagawa 258-8577 (JP)
(74) Representative: Hoffmann Eitle
(86) International application number: PCT/JP2023/043686
(87) International publication number: WO 2024/161783

(57) **Abstract**

A learning support device includes a processor, in which the processor is configured to acquire an original image that is a source of a learning input image for a machine learning model used to ensure monoclonality of a cell seeded in a container, and generate, as the learning input image, an artificial image in which an optical virtual image caused by illumination light onto the container is depicted by performing image processing on the original image.

## Description

### BACKGROUND OF THE INVENTION

### 1. Field of the Invention

The technology of the present disclosure relates to a learning support device, an operation method of a learning support device, and an operation program of a learning support device.

### 2. Description of the Related Art

Recently, there has been an active production of antibody pharmaceuticals by culturing Chinese hamster ovary (hereinafter, referred to as CHO) cells into which an antibody gene is incorporated and causing the CHO cells to produce an antibody. The CHO cells are seeded and cultured, for example, one by one in each of a plurality of wells of a well plate. Then, among the CHO cells in the respective wells, a CHO cell having an excellent antibody production ability (referred to as a stable expression cell line) is selected. In this case, regulatory authorities such as the United States Food and Drug Administration (US FDA) require assurance that one CHO cell is seeded in the well without mistake and that the antibody is produced from one CHO cell without mistake (cellular monoclonality, also referred to as monoclonality).

JP2022-509201A discloses a technology that uses a machine learning model such as a convolutional neural network to ensure the cellular monoclonality. In JP2022-509201A, a captured image of a well is input to the machine learning model, and a plurality of types of objects such as one cell, a doublet (aggregated cells), or debris are extracted from the captured image. In order to train such a machine learning model, in JP2022-509201A, captured images in which any of a plurality of types of objects is shown are collected from a plurality of captured images obtained under a wide variety of imaging conditions such as illuminating various types of wells at different angles.

### SUMMARY OF THE INVENTION

As described above, in JP2022-509201A, in order to train the machine learning model, it is necessary to collect a large number of captured images in which any of a plurality of types of objects is shown while changing the imaging conditions in various ways.

Here, an optical virtual image (artifact) caused by illumination light onto the well is present as an object that should actually be extracted as one cell but is erroneously determined not to be one cell. Specifically, the optical virtual image includes a mirror image that is generated by overlapping a part of one cell and a convergent image that is generated by condensing illumination light due to a lens effect of the cell. In a case in which an image in which such an optical virtual image is shown is to be collected from an actual captured image, it takes a lot of time and effort.

One embodiment according to the technology of the present disclosure provides a learning support device, an operation method of a learning support device, and an operation program of a learning support device capable of training a machine learning model used to ensure the monoclonality of a cell seeded in a container without taking time and effort.

According to the present disclosure, there is provided a learning support device comprising: a processor, in which the processor is configured to acquire an original image that is a source of a learning input image for a machine learning model used to ensure monoclonality of a cell seeded in a container, and generate, as the learning input image, an artificial image in which an optical virtual image caused by illumination light onto the container is depicted by performing image processing on the original image.

It is preferable that the optical virtual image includes a mirror image that is generated by overlapping a part of one cell and a convergent image that is generated by the illumination light being condensed by a lens effect of the cell, and the processor is configured to determine which of the mirror image and the convergent image is to be depicted, according to user's designation or the original image.

It is preferable that the processor is configured to change a parameter that determines an aspect of the optical virtual image.

It is preferable that the optical virtual image includes a mirror image that is generated by overlapping a part of one cell, and the parameter is at least one of a direction of a symmetry axis, a distance of the mirror image from the cell, or a density of the mirror image.

It is preferable that the optical virtual image includes a convergent image that is generated by the illumination light being condensed by a lens effect of the cell, the processor is configured to acquire an image in which the convergent image is shown, as the original image, and set a plurality of reference points on the convergent image shown in the original image, and the parameter is at least one of a movement direction of the reference point or a movement distance of the reference point.

It is preferable that the processor is configured to set the reference point on a boundary between a portion where light is condensed and a portion where light is not condensed in the convergent image shown in the original image.

It is preferable that the processor is configured to change an area ratio between the portion where the light is condensed and the portion where the light is not condensed by moving the reference point according to the parameter.

According to the present disclosure, there is provided an operation method of a learning support device, the operation method comprising: acquiring an original image that is a source of a learning input image for a machine learning model used to ensure monoclonality of a cell seeded in a container; and generating, as the learning input image, an artificial image in which an optical virtual image caused by illumination light onto the container is depicted by performing image processing on the original image.

According to the present disclosure, there is provided an operation program of a learning support device, the operation program causing a computer to execute a process comprising: acquiring an original image that is a source of a learning input image for a machine learning model used to ensure monoclonality of a cell seeded in a container; and generating, as the learning input image, an artificial image in which an optical virtual image caused by illumination light onto the container is depicted by performing image processing on the original image.

According to the technology of the present disclosure, it is possible to provide a learning support device, an operation method of a learning support device, and an operation program of a learning support device capable of training a machine learning model used to ensure the monoclonality of a cell seeded in a container without taking time and effort.

### BRIEF DESCRIPTION OF THE DRAWINGS

Fig. 1 is a diagram showing a state in which CHO cells are seeded in wells of a well plate and the wells are imaged by an imaging device, and a learning support device.
Figs. 2A to 2C are diagrams showing patterns in which a cell is shown, in which Fig. 2A shows a case in which no optical virtual image is generated, Fig. 2B shows a case in which a mirror image is generated, and Fig. 2C shows a case in which a convergent image is generated.
Fig. 3 is a block diagram of a computer constituting the learning support device.
Fig. 4 is a block diagram showing a processing unit of a CPU of the learning support device.
Fig. 5 is a diagram showing processing of a search unit.
Fig. 6 is a diagram showing processing of a generation unit that generates a first artificial image from a first original image.
Fig. 7 is a diagram showing processing of a generation unit that sets a reference point on a boundary between a portion where light is condensed and a portion where light is not condensed in a convergent image shown in a second original image.
Fig. 8 is a diagram showing processing of a generation unit that generates a second artificial image from the second original image.
Fig. 9 is a diagram showing details of the processing of the generation unit that generates the second artificial image from the second original image.
Fig. 10 is a diagram showing details of the processing of the generation unit that generates the second artificial image from the second original image.
Fig. 11 is a diagram showing processing of a learning unit that inputs the first artificial image to an extraction model as a learning input image and trains the extraction model.
Fig. 12 is a diagram showing processing of a learning unit that inputs the second artificial image to an extraction model as a learning input image and trains the extraction model.
Fig. 13 is a diagram showing another example of learning data.
Fig. 14 is a diagram showing another example of learning data.
Fig. 15 is a diagram showing processing of the extraction model in an operation phase.
Fig. 16 is a diagram showing processing of the extraction model in an operation phase.
Fig. 17 is a flowchart showing a processing procedure of the learning support device.
Fig. 18 is a flowchart showing a processing procedure of the learning support device.
Fig. 19 is a flowchart showing a processing procedure of the learning support device.
Fig. 20 is a flowchart showing a processing procedure of the learning support device.
Fig. 21 is a diagram showing an aspect in which a user designates an original image.

### DESCRIPTION OF THE PREFERRED EMBODIMENTS

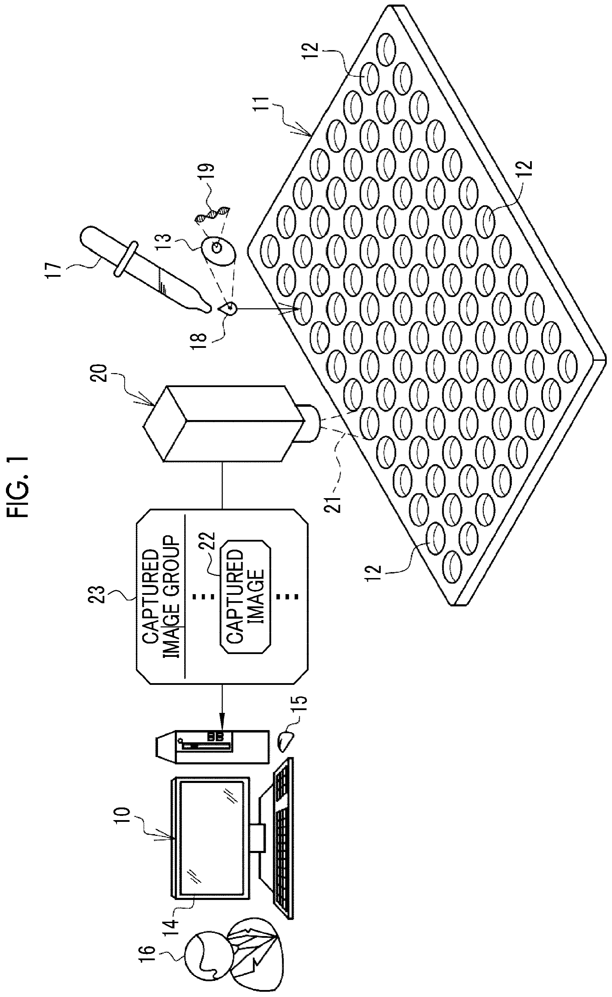

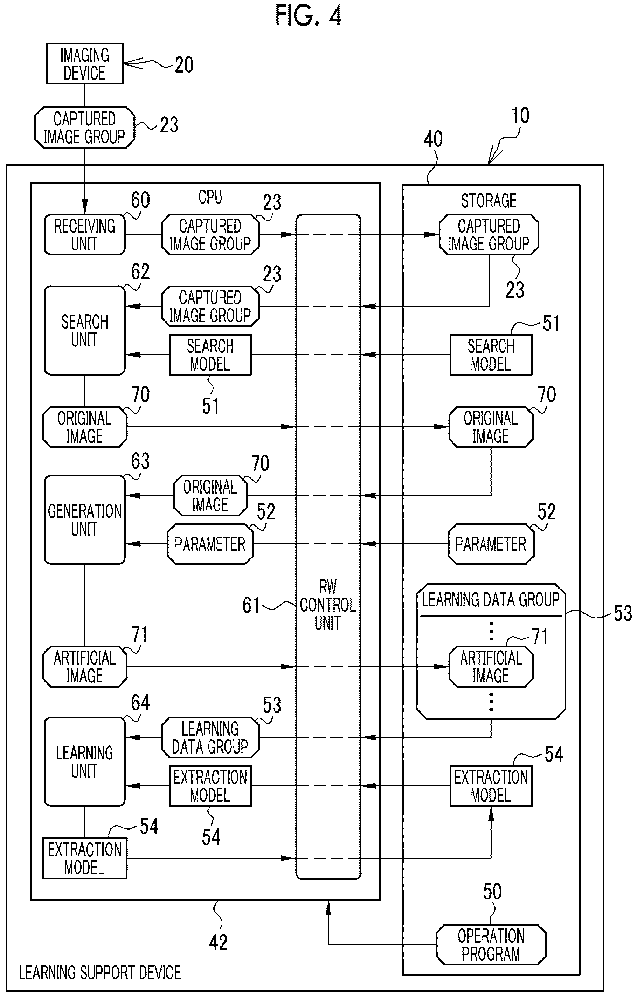

As shown in Fig. 1 as an example, a learning support device 10 according to the technology of the present disclosure supports learning of an extraction model 54 (see Fig. 4) used to ensure the cellular monoclonality of a CHO cell 13 seeded in a well 12 of a well plate 11. The learning support device 10 is, for example, a desktop personal computer and comprises a display 14 that displays various screens and an input device 15 such as a keyboard, a mouse, a touch panel, and/or a microphone for voice input. The learning support device 10 is installed in, for example, a development company of the extraction model 54 and is operated by a user 16 who is involved in the development of the extraction model 54 at the development company.

A plurality of the wells 12 are formed in the well plate 11. Fig. 1 illustrates a so-called 96-well plate in which 12 × 8 = 96 wells 12 are formed. A liquid droplet 18 is dispensed into each well 12 by a pipette 17. The liquid droplet 18 contains a CHO cell 13. An antibody gene 19 is incorporated into the CHO cell 13, and the CHO cell 13 produces an antibody that is a source of antibody pharmaceuticals during a culture process. The well 12 is an example of a "container" according to the technology of the present disclosure. In addition, the CHO cell 13 is an example of a "cell" according to the technology of the present disclosure. Although only one well plate 11 is depicted in Fig. 1, in reality, a large number of well plates 11, for example 10 to 100, are used.

Each well 12 is imaged by an imaging device 20 immediately after the liquid droplet 18 is dispensed. The imaging device 20 is, for example, a digital phase contrast microscope. The digital phase contrast microscope includes a light source, an optical system, an imaging element, and the like. The light source irradiates the well 12 with illumination light 21. The optical system is composed of a plurality of lenses and the like that capture an optical image of the well 12. The imaging element captures an optical image of the well 12 formed by the optical system and outputs a captured image 22 of the well 12. In Fig. 1, it is depicted that one well 12 is imaged in its entirety at once, in reality, one well 12 is divided into a number of regions, each of which is imaged, and images obtained for each region are then combined to generate a captured image 22 in which the entirety of one well 12 is shown.

The imaging device 20 has a function of adjusting the focus of the optical system according to a type of the well plate 11 and a scan result of distortion of a bottom surface of the well 12. Therefore, the captured image 22 is a clear image that is focused on the bottom surface of the well 12 and that has no blurriness. The imaging device 20 transmits a captured image group 23, which is a set of the captured images 22 of the respective wells 12, to the learning support device 10.

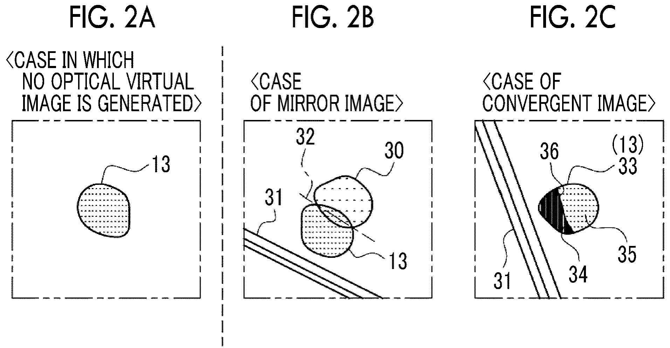

An optical virtual image caused by the illumination light 21 may be generated in the CHO cell 13 shown in the captured image 22. Therefore, as shown in Figs. 2A to 2C as an example, there are three major patterns in which the CHO cell 13 is shown in the captured image 22.

Fig. 2A shows a case in which no optical virtual image caused by the illumination light 21 is generated. In this case, the CHO cell 13 has a clear contour and almost no shading in density (optical density). Therefore, it is possible to extract one CHO cell 13 as it is with high reliability.

On the other hand, Figs. 2B and 2C show a case in which an optical virtual image caused by the illumination light 21 is generated. Fig. 2B shows a mirror image 30 that is generated by overlapping a part of one CHO cell 13. The mirror image 30 is generated in the vicinity of a side wall 31 of the well 12. The mirror image 30 has a line that passes through a part of the CHO cell 13 and that is parallel to the side wall 31, as a symmetry axis 32. In other words, the mirror image 30 is an image generated by folding an image of the CHO cell 13 at the symmetry axis 32.

Here, the side wall 31 is a curve in a macroscopic view, but is a straight line in a microscopic view. Therefore, the symmetry axis 32 is expressed as "a line parallel to the side wall 31" as described above. Here, the term "parallel" refers to parallel in a meaning including an error that is generally allowed in the technical field to which the technology of the present disclosure belongs and that does not contradict the gist of the technology of the present disclosure, in addition to completely parallel.

Fig. 2C shows a convergent image 33 that is generated by the illumination light 21 being condensed by a lens effect of the CHO cell 13. The convergent image 33 is likely to be generated in the vicinity of the side wall 31, but is also generated at a location other than the vicinity of the side wall 31. The convergent image 33 is divided into a portion where light is condensed (hereinafter, referred to as a condensed portion) 34 and a portion where light is not condensed (hereinafter, referred to as a non-condensed portion) 35. The condensed portion 34 is a portion with a relatively high density and appears whitish. Conversely, the non-condensed portion 35 is a portion with a relatively low density and appears dark. Therefore, a boundary 36 appears between the condensed portion 34 and the non-condensed portion 35.

The mirror image 30 shown in Fig. 2B and the convergent image 33 shown in Fig. 2C are difficult to extract one CHO cell 13 as it is, as compared with the general appearance of the CHO cell 13 in the case of Fig. 2A. Therefore, the CHO cell 13 is overlooked without being extracted as one CHO cell 13, and as a result, an erroneous determination that only one CHO cell 13 is seeded is made even though a plurality of CHO cells 13 are actually seeded. Therefore, in a learning phase of the extraction model 54, it is necessary to perform intensive learning for the mirror image 30 and the convergent image 33.

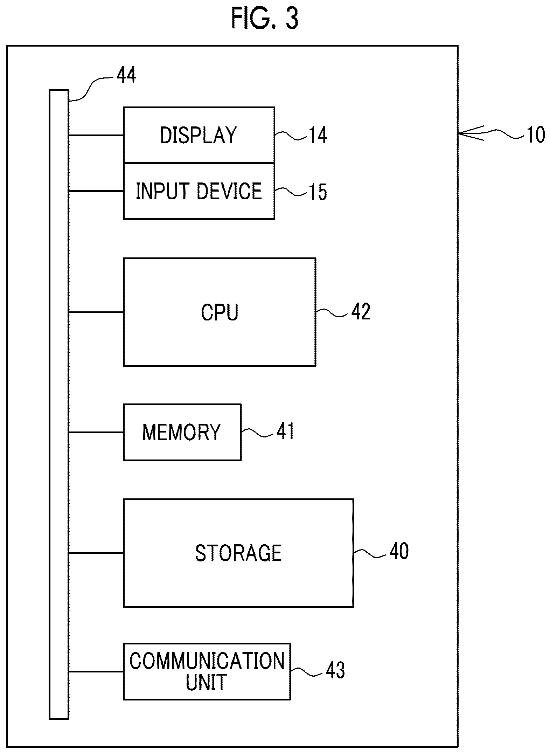

As shown in Fig. 3 as an example, a computer constituting the learning support device 10 comprises a storage 40, a memory 41, a central processing unit (CPU) 42, and a communication unit 43 in addition to the display 14 and the input device 15 described above. These are interconnected via a busline 44.

The storage 40 is a hard disk drive that is built into the computer constituting the learning support device 10 or that is connected via a cable or a network. Alternatively, the storage 40 is a disk array in which a plurality of hard disk drives are connected in series. The storage 40 stores a control program such as an operating system, various application programs, various data associated with these programs, and the like. A solid state drive may be used instead of the hard disk drive.

The memory 41 is a work memory for the CPU 42 to execute processing. The CPU 42 loads the program stored in the storage 40 into the memory 41 and executes processing corresponding to the program. Thus, the CPU 42 collectively controls the respective units of the computer. The CPU 42 is an example of a "processor" according to the technology of the present disclosure. The memory 41 may be built into the CPU 42. The communication unit 43 controls the transmission of various types of information to an external device such as the imaging device 20.

As shown in Fig. 4 as an example, an operation program 50 is stored in the storage 40 of the learning support device 10. The operation program 50 is an application program for causing the computer to function as the learning support device 10. That is, the operation program 50 is an example of "an operation program of a learning support device" according to the technology of the present disclosure. The storage 40 also stores a search model 51, a parameter 52, a learning data group 53, an extraction model 54, and the like. The extraction model 54 is an example of a "machine learning model" according to the technology of the present disclosure.

In a case in which the operation program 50 is activated, the CPU 42 of the computer constituting the learning support device 10 functions as a receiving unit 60, a read/write (hereinafter, referred to as RW) control unit 61, a search unit 62, a generation unit 63, and a learning unit 64 in cooperation with the memory 41 and the like.

The receiving unit 60 receives the captured image group 23 from the imaging device 20. The receiving unit 60 outputs the received captured image group 23 to the RW control unit 61.

The RW control unit 61 controls the read-out of various data stored in the storage 40 and the storage of various data in the storage 40. For example, the RW control unit 61 stores the captured image group 23 from the receiving unit 60 in the storage 40.

The RW control unit 61 reads out the captured image group 23 and the search model 51 from the storage 40, and outputs the read-out captured image group 23 and search model 51 to the search unit 62. In addition, the RW control unit 61 reads out the parameter 52 from the storage 40, and outputs the read-out parameter 52 to the generation unit 63. Further, the RW control unit 61 reads out the learning data group 53 and the extraction model 54 from the storage 40, and outputs the read out learning data group 53 and extraction model 54 to the learning unit 64.

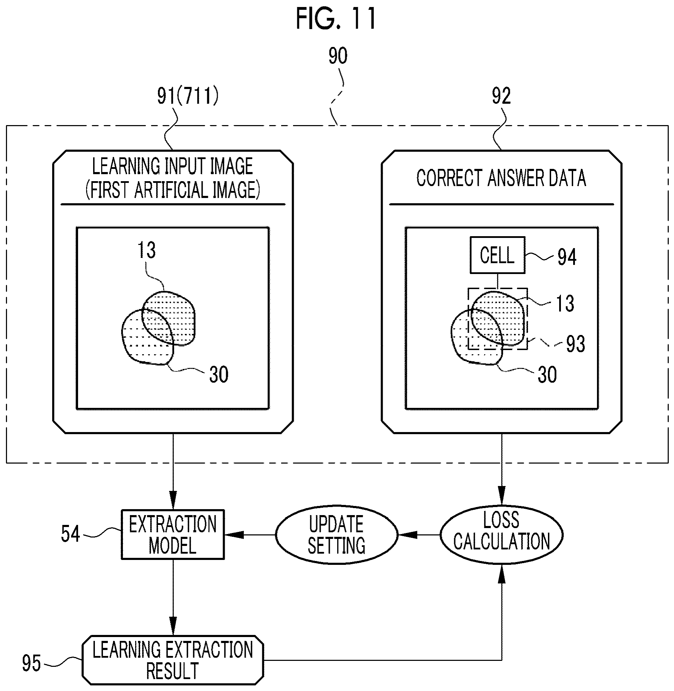

The search unit 62 uses the search model 51 to search for an original image 70 from among the captured images 22 of the captured image group 23. The original image 70 is an image that is a source of an artificial image 71 as a learning input image 91 (see Fig. 11) for the extraction model 54. The search unit 62 outputs the searched original image 70 to the RW control unit 61.

The RW control unit 61 stores the original image 70 from the search unit 62 in the storage 40. In addition, the RW control unit 61 reads out the original image 70 from the storage 40, and outputs the read-out original image 70 to the generation unit 63 together with the parameter 52.

The generation unit 63 performs image processing on the original image 70 according to the parameter 52, thereby generating an artificial image 71 in which an optical virtual image of either the mirror image 30 or the convergent image 33 is depicted. The parameter 52 is data that determines an aspect of the optical virtual image depicted in the artificial image 71. The generation unit 63 outputs the generated artificial image 71 to the RW control unit 61.

The RW control unit 61 stores the artificial image 71 from the generation unit 63 in the learning data group 53 of the storage 40 as the learning input image 91 for the extraction model 54. As a result, the learning data group 53 includes the artificial image 71.

The learning data group 53 is configured of a plurality of learning data 90 (see Fig. 11). The learning data 90 is a set of the learning input image 91 and correct answer data 92 (see Fig. 11). The learning unit 64 trains the extraction model 54 by using the learning data 90. The learning unit 64 outputs the trained extraction model 54 to the RW control unit 61.

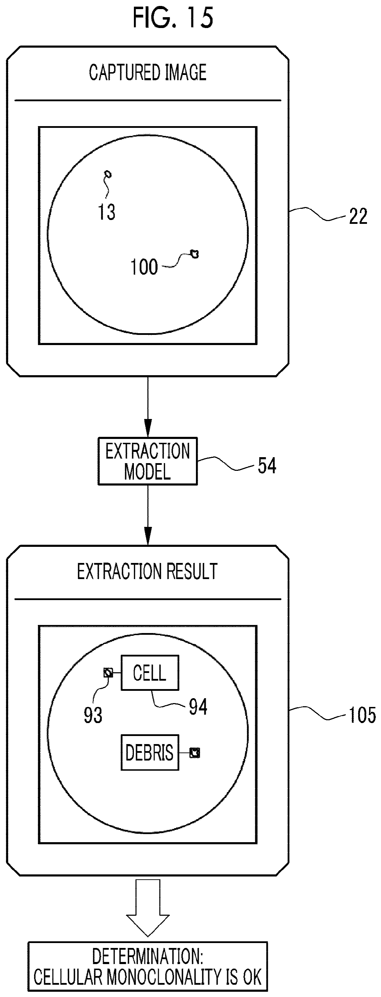

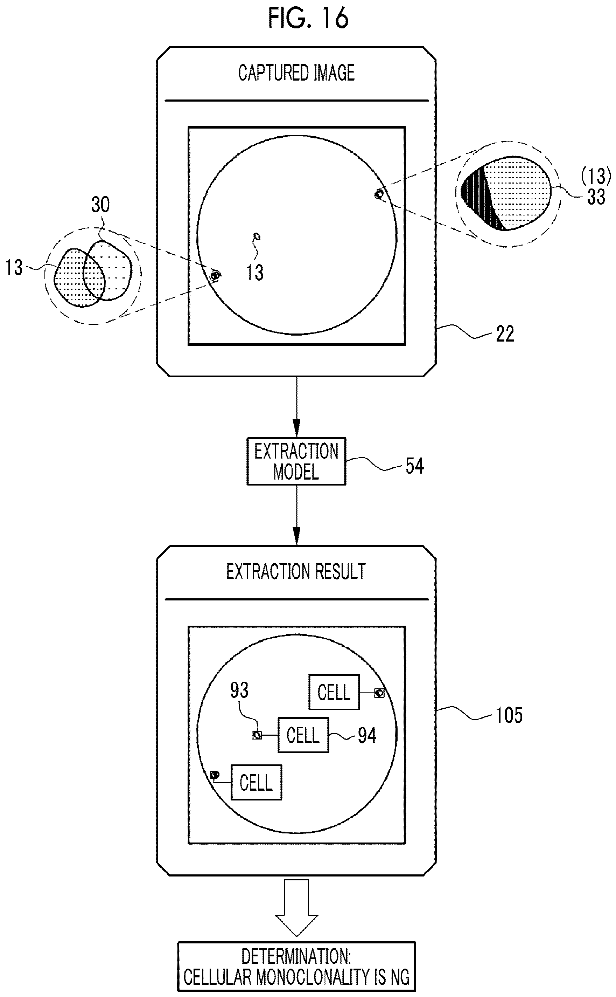

The RW control unit 61 stores the trained extraction model 54 from the learning unit 64 in the storage 40. The trained extraction model 54 is used to ensure the cellular monoclonality of the CHO cell 13 (see Figs. 15 and 16). The trained extraction model 54 may be stored in a removable medium such as a compact disc recordable (CD-R) or a universal serial bus (USB) memory, and the trained extraction model 54 may be transferred to a device other than the learning support device 10.

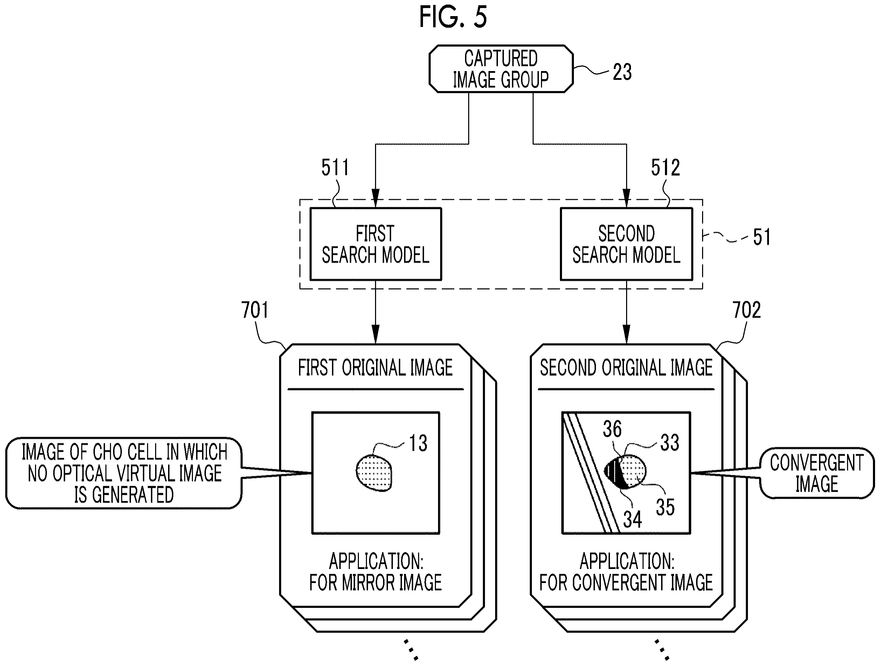

As shown in Fig. 5 as an example, the search model 51 is composed of a first search model 511 and a second search model 512. The search unit 62 inputs the captured image group 23 to each of the first search model 511 and the second search model 512. Then, the first search model 511 outputs a first original image 701, and the second search model 512 outputs a second original image 702. The first search model 511 and the second search model 512 are machine learning models, such as a convolutional neural network, that are responsible for a task of searching for the first original image 701 and the second original image 702 from among the captured images 22 of the captured image group 23.

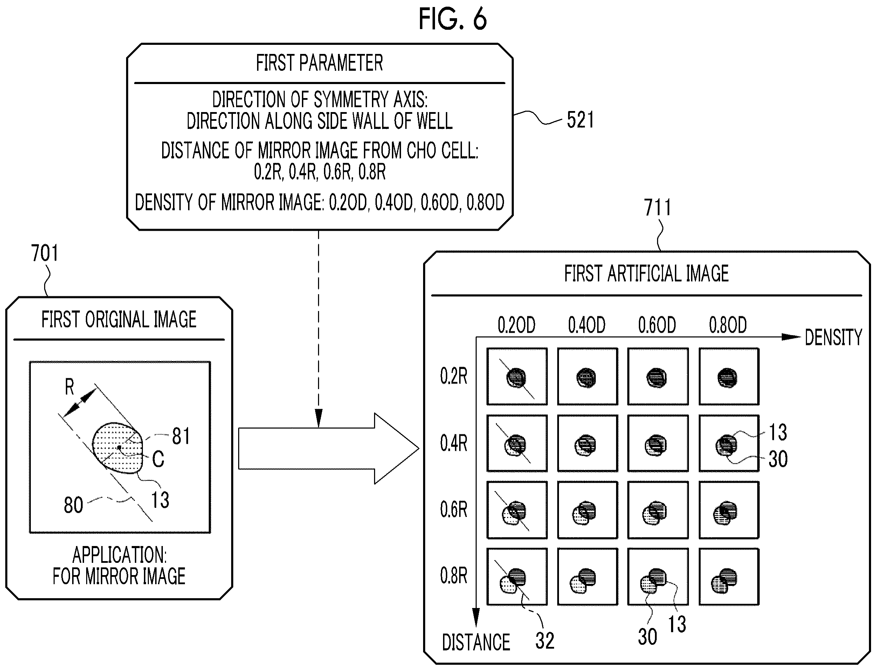

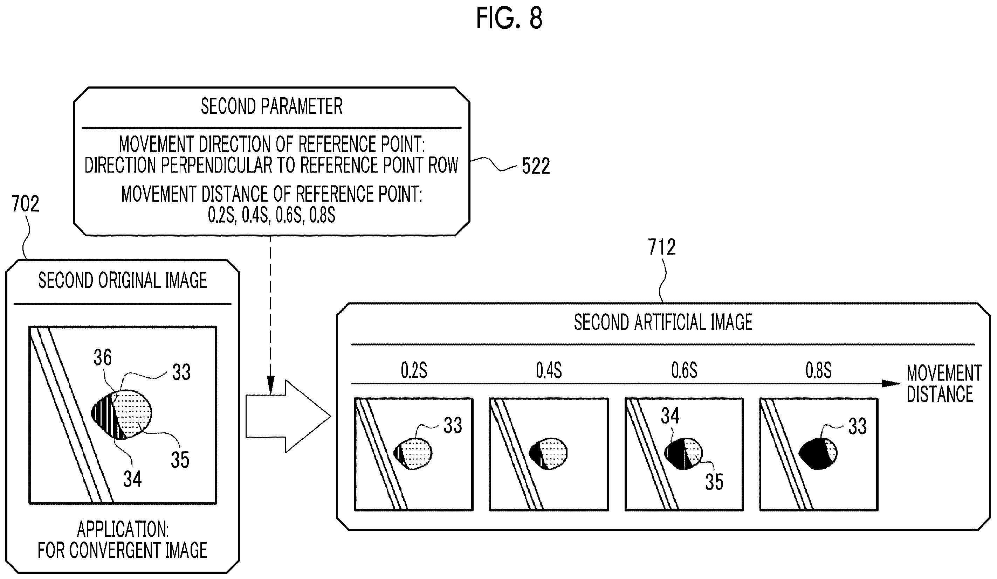

The first original image 701 is an image of the CHO cell 13 shown in Fig. 2A in which no optical virtual image is generated. The first original image 701 is used for the mirror image 30, and the first original image 701 is used for generating a first artificial image 711 (see Fig. 6). On the other hand, the second original image 702 is the convergent image 33 shown in Fig. 2C. The second original image 702 is used for the convergent image 33, and the second original image 702 is used for generating a second artificial image 712 (see Fig. 8).

In Fig. 5, a case in which both the first original image 701 and the second original image 702 are searched for a plurality of times is illustrated, but at least one of the first original image 701 or the second original image 702 need only be searched for. In addition, in a case in which the arrangement of the captured image group 23 is poor, none of the first original image 701 and/or the second original image 702 may be searched for. In such a case, the captured image group 23 need only be changed to another one to search for the first original image 701 and/or the second original image 702 again.

As shown in Fig. 6 as an example, in a case in which the first original image 701 is used for the mirror image 30, the generation unit 63 generates a first artificial image 711 in which the mirror image 30 is depicted by performing image processing on the first original image 701 according to a first parameter 521. The first parameter 521 is a direction of a symmetry axis, a distance of the mirror image 30 from the CHO cell 13, and a density of the mirror image 30. Here, as the direction of the symmetry axis, a direction (direction of a line 80) along the side wall 31 of the well 12 is set. In addition, four types of distances of 0.2R, 0.4R, 0.6R, and 0.8R are set as the distance of the mirror image 30. Further, four types of densities of 0.2OD, 0.4OD, 0.6OD, and 0.8OD are set as the density of the mirror image 30.

Information on the line 80 along the side wall 31 can be obtained by analyzing the original captured image 22 of the first original image 701. The distance of the mirror image 30 from the CHO cell 13 is specifically a distance from a center C of the CHO cell 13 to a center of the mirror image 30. R is a length of a perpendicular line 81 to the line 80, which passes through the center C of the CHO cell 13 in the first original image 701 and that has two opposite points on a contour line of the CHO cell 13 as a start point and an end point. R may be rephrased as a diameter of the CHO cell 13. A value that is larger than 0 and smaller than R is set as the distance of the mirror image 30 from the CHO cell 13. The reason for setting the distance of the mirror image 30 from the CHO cell 13 to a value smaller than R is that, in a case in which the distance is set to a value equal to or larger than R, the mirror image 30 does not overlap a part of the CHO cell 13 and appears as two CHO cells 13. OD is an average value of the densities of the CHO cells 13 in the first original image 701. A lower limit value of the density of the mirror image 30 is, for example, 0.1OD, and an upper limit value thereof is, for example, 0.9OD.

The generation unit 63 sets the symmetry axis 32 along the side wall 31 at a position corresponding to the distance of the first parameter 521 for the CHO cell 13 shown in the first original image 701. The generation unit 63 folds the image of the CHO cell 13 at the symmetry axis 32 to depict the mirror image 30, and then changes the density of the mirror image 30 according to the density of the first parameter 521. As a result, the first artificial image 711 is generated. In Fig. 6, since four types of distances are set as the distance of the mirror image 30 from the CHO cell 13 and four types of densities are set as the density of the mirror image 30, 4 × 4 = 16 first artificial images 711 are generated from one first original image 701.

Although the direction of the symmetry axis 32 is set to one type, the present disclosure is not limited to this, and a plurality of types of directions of the symmetry axis 32 may be set, such as the distance of the mirror image 30 from the CHO cell 13. In addition, the distance of the mirror image 30 from the CHO cell 13 and the density of the mirror image 30 are not limited to the four types of examples, and may be two types or five or more types. For example, as the distance of the mirror image 30 from the CHO cell 13, nine types of 0.1R, 0.2R, 0.3R, 0.4R, 0.5R, 0.6R, 0.7R, 0.8R, and 0.9R may be set.

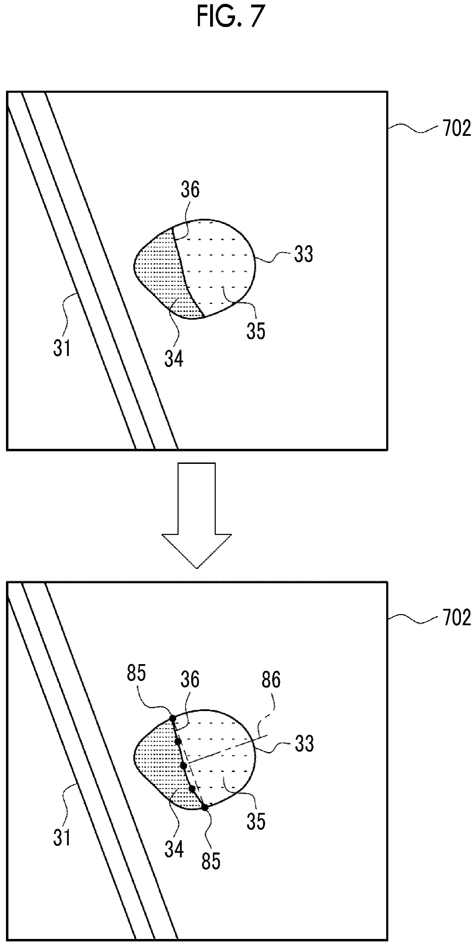

As shown in Fig. 7 as an example, in a case in which the second original image 702 is used for the convergent image 33, the generation unit 63 sets a plurality of reference points 85 on the convergent image 33 shown in the second original image 702. More specifically, the generation unit 63 sets the plurality of reference points 85 at equal intervals on the boundary 36 between the condensed portion 34 and the non-condensed portion 35 of the convergent image 33.

As shown in Fig. 8 as an example, the generation unit 63 generates a second artificial image 712 in which the convergent image 33 is depicted by performing image processing on the second original image 702 according to a second parameter 522. The second parameter 522 is a movement direction of the reference point 85 and a movement distance of the reference point 85. Here, a direction perpendicular to a reference point row is set as the movement direction of the reference point 85. In addition, four types of movement distances of 0.2S, 0.4S, 0.6S, and 0.8S are set as the movement distance of the reference point 85.

The direction perpendicular to the reference point row is a direction of a perpendicular line 86 (see Fig. 7) of a line connecting two reference points 85 set at both ends of the boundary 36. S is an area of the CHO cell 13 in the second original image 702. 0.2S, 0.4S, and the like indicate an area ratio between the condensed portion 34 and the non-condensed portion 35. For example, 0.2S means that the area of the condensed portion 34 accounts for 20% of the area of the CHO cell 13 (the area of the non-condensed portion 35 accounts for 80% of the area of the CHO cell 13). Therefore, the movement distance of the reference point 85 may be rephrased as the area ratio between the condensed portion 34 and the non-condensed portion 35. A value that is larger than 0 and smaller than S is set as the movement distance of the reference point 85.

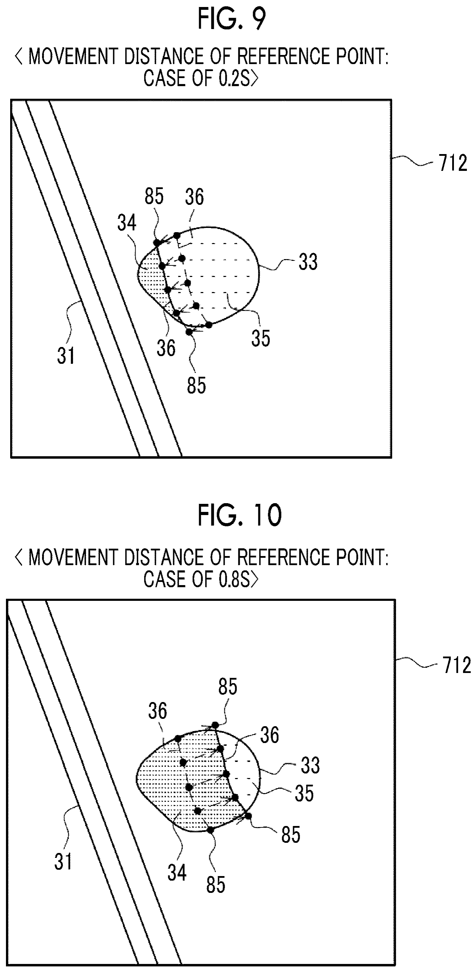

As shown in Figs. 9 and 10 as an example, the generation unit 63 changes the area ratio between the condensed portion 34 and the non-condensed portion 35 by moving the reference point 85 set on the boundary 36 according to the movement direction and the movement distance of the second parameter 522. Fig. 9 shows a case in which the movement distance of the reference point 85 is 0.2S, and Fig. 10 shows a case in which the movement distance of the reference point 85 is 0.8S. As a result, the second artificial image 712 is generated. In Fig. 8, since four types of movement distances are set as the movement distance of the reference point 85, four second artificial images 712 are generated from one second original image 702.

Although the movement direction of the reference point 85 is set to one type, the present disclosure is not limited to this, and a plurality of types of movement directions of the reference point 85 may be set. In addition, the movement distance of the reference point 85 is not limited to the four types of examples, and may be two types or five or more types. For example, as the movement distance of the reference point 85, nine types of 0.1S, 0.2S, 0.3S, 0.4S, 0.5S, 0.6S, 0.7S, 0.8S, and 0.9S may be set.

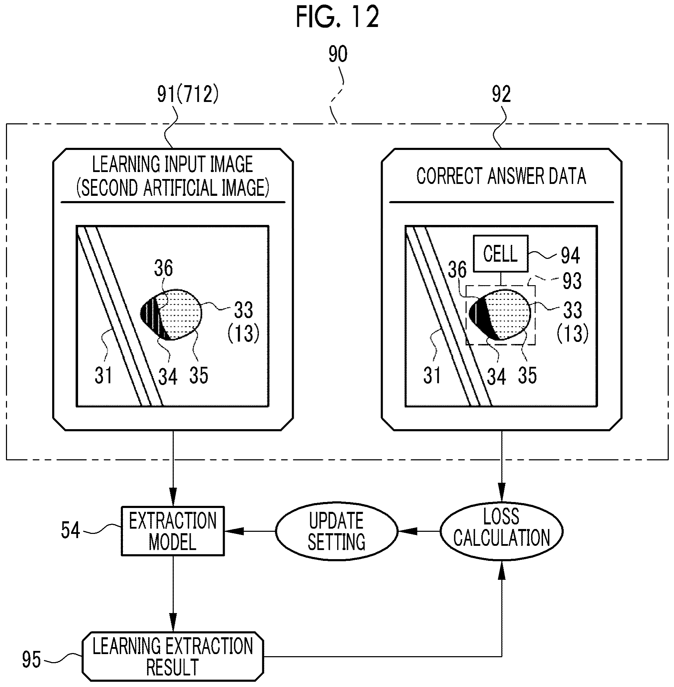

As shown in Figs. 11 and 12 as an example, the learning unit 64 trains the extraction model 54 by using the learning data 90. The learning data 90 is a set of the learning input image 91 and the correct answer data 92 as described above. The correct answer data 92 is annotated with a bounding box 93, which is a square-shaped frame surrounding the object shown in the learning input image 91, and a class 94 of the object.

The learning unit 64 inputs the learning input image 91 in the learning data 90 to the extraction model 54, and causes the extraction model 54 to output a learning extraction result 95. The learning unit 64 compares the correct answer data 92 with the learning extraction result 95, and performs loss calculation of the extraction model 54 using a loss function based on the comparison result. The learning unit 64 performs update setting of coefficients of the extraction model 54 according to the result of the loss calculation, and updates the extraction model 54 according to the update setting.

The learning unit 64 repeatedly performs the above series of processes of inputting the learning input image 91 to the extraction model 54, outputting the learning extraction result 95 from the extraction model 54, performing the loss calculation, performing the update setting, and updating the extraction model 54, while changing the learning data 90. In a case in which the extraction accuracy of the learning extraction result 95 for the correct answer data 92 reaches a preset level, the learning unit 64 ends the repetition of the series of processes. The learning unit 64 outputs the extraction model 54 of which the extraction accuracy has reached the preset level to the RW control unit 61 as the trained extraction model 54. Regardless of the extraction accuracy, the learning may be ended in a case in which the series of processes has been repeated a predetermined number of times.

Fig. 11 shows a case in which the first artificial image 711 is used as the learning input image 91. In this case, the correct answer data 92 is data annotated with the bounding box 93 surrounding the CHO cell 13 adjacent to the mirror image 30 as an object and the "cell" as the class 94. On the other hand, Fig. 12 shows a case in which the second artificial image 712 is used as the learning input image 91. In this case, the correct answer data 92 is data annotated with the bounding box 93 surrounding the CHO cell 13 of the convergent image 33 as an object and the "cell" as the class 94. For convenience of description, the object is drawn in the correct answer data 92, but the actual correct answer data 92 only has information on the position and the size of the bounding box 93 and the class 94.

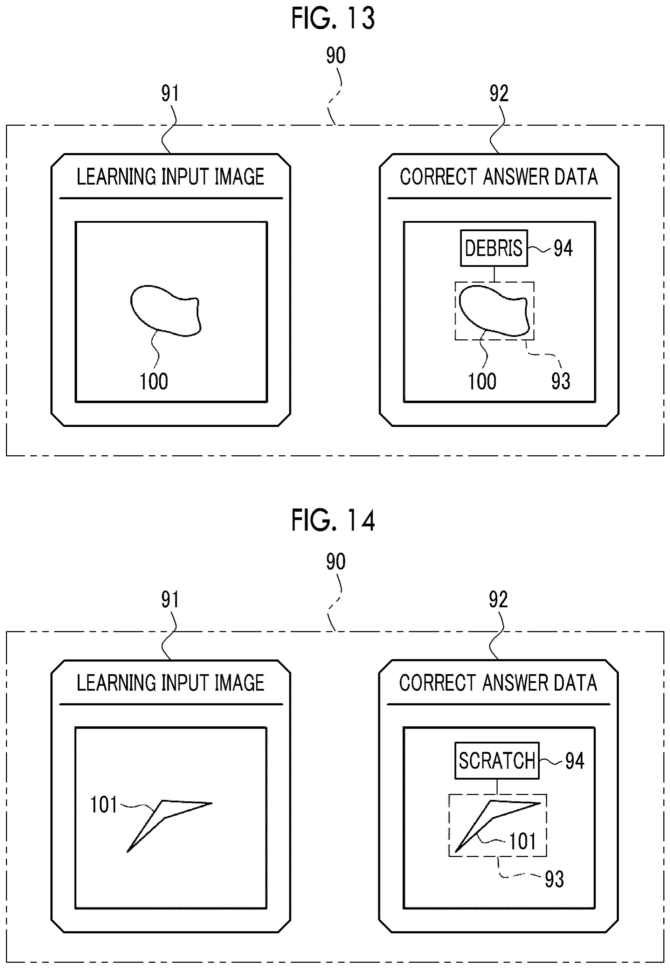

Figs. 13 and 14 show another example of the learning data 90. The learning data 90 shown in Fig. 13 as an example includes a learning input image 91 in which debris 100 such as dust and dirt is captured as an object. In this case, the correct answer data 92 is data annotated with the bounding box 93 surrounding the debris 100 as an object and the "debris" as the class 94. The learning data 90 shown in Fig. 14 as an example includes a learning input image 91 in which a scratch 101 formed on the bottom surface of the well 12 is captured as an object. In this case, the correct answer data 92 is data annotated with the bounding box 93 surrounding the scratch 101 as an object and the "scratch" as the class 94. Although not shown, there is of course also learning data 90 including, as the learning input image 91, the image of the CHO cell 13 in which no optical virtual image is generated, as shown in Fig. 2A. As described above, the learning data 90 may or may not include the first artificial image 711 and the second artificial image 712 as the learning input image 91. By performing learning using such a wide variety of learning data 90, the extraction model 54 becomes a model that extracts various objects shown in the captured image 22, attaches the bounding box 93 and the class 94 to the extracted object, and outputs the extracted object as an extraction result 105 (see Fig. 15).

As shown in Figs. 15 and 16 as an example, in an operation phase of the extraction model 54, the captured image 22 in which the shown object is unknown is input to the extraction model 54. In response to this, the extraction result 105 is output from the extraction model 54.

Fig. 15 illustrates a case in which the captured image 22 in which one CHO cell 13 in which no optical virtual image is generated and one debris 100 are shown as objects is input. In this case, the extraction result 105 is such that the bounding box 93 surrounding the CHO cell 13 and the class 94 of "cell", and the bounding box 93 surrounding the debris 100 and the class 94 of "debris" are added. In this case, since the number of the extracted CHO cells 13 is one, the user 16 determines that the cellular monoclonality is OK.

Fig. 16 illustrates a case in which the captured image 22 in which one CHO cell 13 in which no optical virtual image is generated, one CHO cell 13 in which the mirror image 30 is generated, and one CHO cell 13 in which the convergent image 33 is generated are shown as objects is input. In this case, the extraction result 105 is such that the bounding box 93 surrounding each of the CHO cell 13 in which no optical virtual image is generated, the CHO cell 13 in which the mirror image 30 is generated, and the CHO cell 13 in which the convergent image 33 is generated is added. In addition, the extraction result 105 in this case is such that the class 94 of "cell" is added to each of the CHO cell 13 in which no optical virtual image is generated, the CHO cell 13 in which the mirror image 30 is generated, and the CHO cell 13 in which the convergent image 33 is generated. In this case, since the number of the extracted CHO cells 13 is not one, the user 16 determines that the cellular monoclonality is No Good (NG). The operation of the extraction model 54 may be performed by the learning support device 10 or may be performed by a device other than the learning support device 10. In addition, even after the operation of the extraction model 54, the learning using the learning data 90 may be continued.

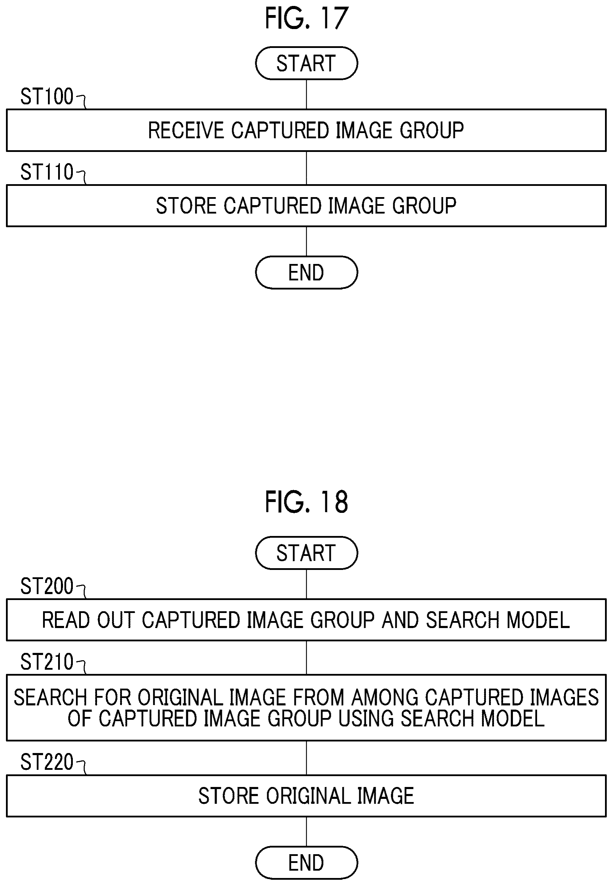

Next, an operation of the configuration described above will be described with reference to flowcharts shown in Figs. 17 to 20 as an example. In a case in which the operation program 50 is activated in the learning support device 10, as shown in Fig. 4, the CPU 42 of the learning support device 10 functions as the receiving unit 60, the RW control unit 61, the search unit 62, the generation unit 63, and the learning unit 64.

First, the receiving unit 60 receives the captured image group 23 from the imaging device 20 (step ST100 in Fig. 17). The captured image group 23 is output from the receiving unit 60 to the RW control unit 61 and are stored in the storage 40 under the control of the RW control unit 61 (step ST110).

The captured image group 23 and the search model 51 are read out from the storage 40 by the RW control unit 61 (step ST200 in Fig. 18). The captured image group 23 and the search model 51 are output from the RW control unit 61 to the search unit 62.

In the search unit 62, as shown in Fig. 5, the original image 70 is searched for from among the captured images 22 of the captured image group 23 using the search model 51 (step ST210). More specifically, the first original image 701 for the mirror image 30 is searched for by the first search model 511, and the second original image 702 for the convergent image 33 is searched for by the second search model 512. The original image 70 is output from the search unit 62 to the RW control unit 61 and are stored in the storage 40 under the control of the RW control unit 61 (step ST220).

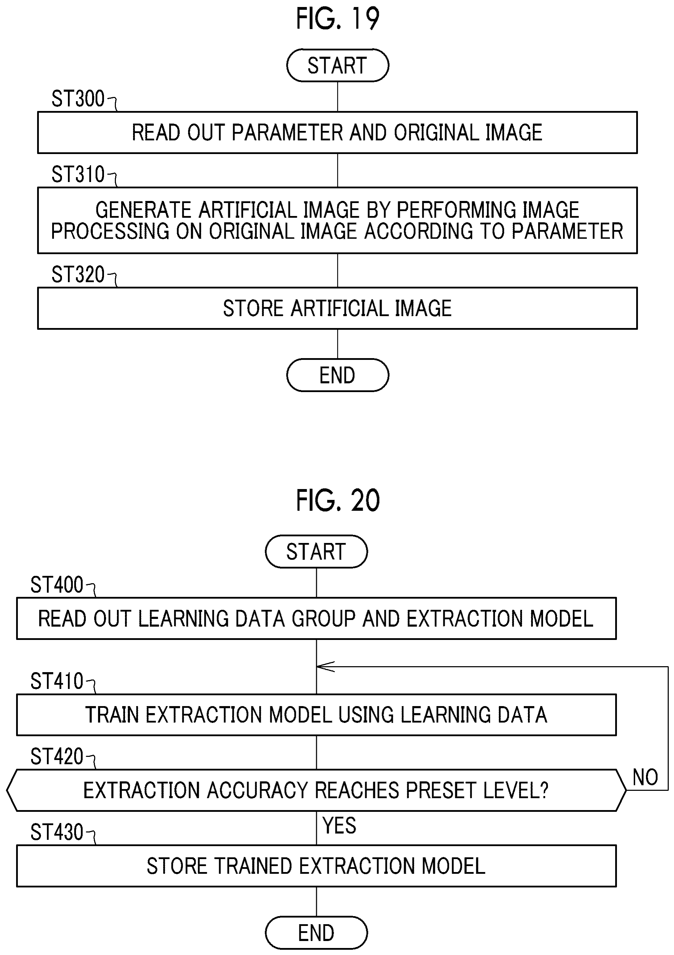

The parameter 52 and the original image 70 are read out from the storage 40 by the RW control unit 61 (step ST300 in Fig. 19). The parameter 52 and the original image 70 are output from the RW control unit 61 to the generation unit 63.

In the generation unit 63, as shown in Figs. 6 to 10, the artificial image 71 is generated by performing the image processing on the original image 70 according to the parameter 52 (step ST310). More specifically, as shown in Fig. 6, the first artificial image 711 in which the mirror image 30 is depicted is generated by performing the image processing on the first original image 701 according to the first parameter 521. In addition, as shown in Figs. 7 to 10, the second artificial image 712 in which the convergent image 33 is depicted is generated by performing the image processing on the second original image 702 according to the second parameter 522. The artificial image 71 is output from the generation unit 63 to the RW control unit 61 and are stored in the storage 40 as a part of the learning data 90 under the control of the RW control unit 61 (step ST320).

The learning data group 53 and the extraction model 54 are read out from the storage 40 by the RW control unit 61 (step ST400 in Fig. 20). The learning data group 53 and the extraction model 54 are output from the RW control unit 61 to the learning unit 64.

In the learning unit 64, as shown in Figs. 11 and 12, the extraction model 54 is trained using the learning data 90 (step ST410). The process of step ST410 is repeatedly performed while the learning data 90 is changed until the extraction accuracy of the learning extraction result 95 with respect to the correct answer data 92 reaches a preset level (NO in step ST420).

In a case in which the extraction accuracy reaches a preset level (YES in step ST420), the repetition of the process of step ST410 is ended. The extraction model 54 is output from the learning unit 64 to the RW control unit 61 and is stored in the storage 40 as the trained extraction model 54 under the control of the RW control unit 61 (step ST430).

As described above, the CPU 42 of the learning support device 10 comprises the search unit 62 and the generation unit 63. The search unit 62 acquires the original image 70 by searching for the original image 70 from among the captured images 22 of the captured image group 23. The original image 70 is an image that is a source of the learning input image 91 of the extraction model 54 used to ensure the cellular monoclonality of the CHO cell 13 seeded in the well 12. The generation unit 63 generates the artificial image 71 in which the optical virtual image caused by the illumination light 21 onto the well 12 is depicted by performing image processing on the original image 70, as the learning input image 91. Therefore, it is possible to save the time and effort to collect the captured images 22 in which the optical virtual image is shown. Therefore, it is possible to train the extraction model 54 without taking time and effort.

As shown in Figs. 2A to 2C, the optical virtual image includes the mirror image 30 that is generated by overlapping a part of one CHO cell 13 and the convergent image 33 that is generated by the illumination light 21 being condensed by the lens effect of the CHO cell 13. As shown in Figs. 6 and 8, in a case in which the first original image 701 is used, the generation unit 63 generates the first artificial image 711 in which the mirror image 30 is depicted. On the other hand, in a case in which the second original image 702 is used, the generation unit 63 generates the second artificial image 712 in which the convergent image 33 is depicted. That is, the generation unit 63 determines which of the mirror image 30 and the convergent image 33 is to be depicted on the artificial image 71, according to the original image 70. Therefore, the artificial image 71 corresponding to the original image 70 can be generated.

As shown in Figs. 6 and 8, the generation unit 63 can change the parameter 52 that determines the aspect of the optical virtual image. Therefore, a plurality of the artificial images 71 can be generated from one original image 70, and the learning data 90 can be further enriched. As a result, the learning of the extraction model 54 progresses, and the extraction accuracy of the extraction model 54 can be improved.

As shown in Fig. 6, the first parameter 521 in a case of the mirror image 30 is the direction of the symmetry axis, the distance of the mirror image 30 from the CHO cell 13, and the density of the mirror image 30. Therefore, it is possible to generate, from one first original image 701, a wide variety of first artificial images 711 in which the directions of the symmetry axes are different, the distances of the mirror images 30 from the CHO cells 13 are different, or the densities of the mirror images 30 are different. As a result, the learning data 90 can be further enriched. As a result, the learning of the extraction model 54 progresses, and the extraction accuracy of the extraction model 54 can be improved. The first parameter 521 need only be at least one of the direction of the symmetry axis, the distance of the mirror image 30 from the CHO cell 13, or the density of the mirror image 30.

As shown in Fig. 5, the search unit 62 acquires the image in which the convergent image 33 is shown by searching for the image as the second original image 702. As shown in Fig. 7, the generation unit 63 sets the plurality of reference points 85 on the convergent image 33 shown in the second original image 702. As shown in Fig. 8, the second parameter 522 in a case of the convergent image 33 is the movement direction of the reference point 85 and the movement distance of the reference point 85. Therefore, it is possible to generate, from one second original image 702, a wide variety of second artificial images 712 in which the movement directions of the reference points 85 are different or the movement distances of the reference points 85 are different. As a result, the learning data 90 can be further enriched. As a result, the learning of the extraction model 54 progresses, and the extraction accuracy of the extraction model 54 can be improved. The second parameter 522 need only be at least one of the movement direction of the reference point 85 or the movement distance of the reference point 85.

As shown in Fig. 7, the generation unit 63 sets the reference point 85 on the boundary 36 between the condensed portion 34 and the non-condensed portion 35 of the convergent image 33 shown in the second original image 702. Then, as shown in Figs. 9 and 10, the area ratio between the condensed portion 34 and the non-condensed portion 35 is changed by moving the reference point 85 according to the second parameter 522. Therefore, it is possible to generate, from one second original image 702, the second artificial images 712 having various different area ratios between the condensed portion 34 and the non-condensed portion 35, thereby further enriching the learning data 90. As a result, the learning of the extraction model 54 progresses, and the extraction accuracy of the extraction model 54 can be improved.

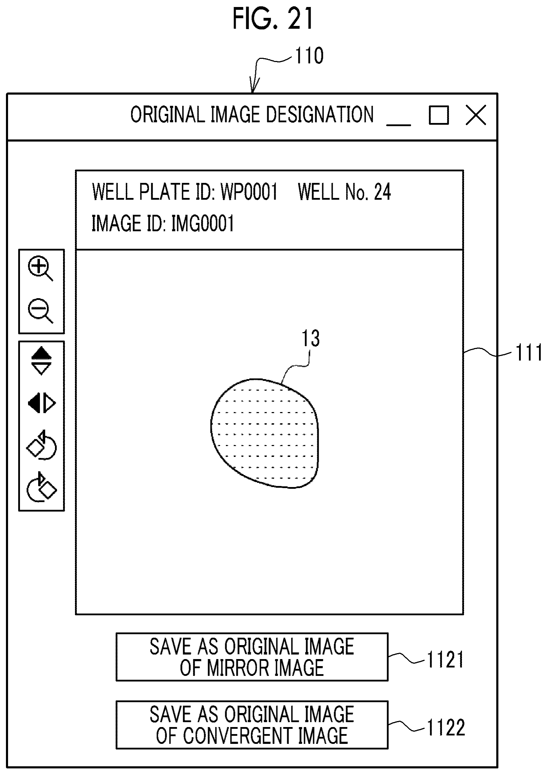

The user 16 may manually search for the original image 70 without using the search model 51. In this case, an original image designation screen 110 shown in Fig. 21 as an example is displayed on the display 14. The original image designation screen 110 has an image display region 111. In the image display region 111, one captured image 22 selected by the user 16 among the plurality of captured images 22 of the captured image group 23 is displayed. In the image display region 111, the captured image 22 can be enlarged, reduced, moved, and rotated.

The original image designation screen 110 is provided with a first save button 1121 and a second save button 1122. The first save button 1121 is a button for saving the image displayed in the image display region 111 as the first original image 701. The second save button 1122 is a button for saving the image displayed in the image display region 111 as the second original image 702.

In this case, the generation unit 63 determines which of the mirror image 30 and the convergent image 33 is to be depicted on the artificial image 71, according to the designation of the user 16. As a result, the artificial image 71 can be generated according to the designation of the user 16.

The parameter 52 may be configured to be freely set by the user 16. Similarly, the reference point 85 may also be configured to be freely set by the user 16.

In the above-described embodiment, it has been described that the CHO cell 13 is newly seeded in the well 12 and the captured image 22 is captured by the imaging device 20 as if the acquisition of the original image 70 is intended, but the present disclosure is not limited to this. The original image 70 may be acquired from the captured image 22 that has been captured in the past and already stored in the storage 40. In addition, the original image 70 may be acquired from an image attached to a paper published on the Internet, instead of the captured image 22 actually captured by the imaging device 20 by the user 16.

An aspect in which the learning support device 10 trains the extraction model 54 has been described, but the present disclosure is not limited to this. The learning support device 10 may only generate the artificial image 71, and a device other than the learning support device 10 may perform the learning of the extraction model 54.

An extraction model that is specialized in extracting one object, such as an extraction model that exclusively extracts the CHO cell 13 in which the mirror image 30 is generated, or an extraction model that exclusively extracts the CHO cell 13 in which the convergent image 33 is generated, may be used.

The hardware configuration of the computer constituting the learning support device 10 according to the technology of the present disclosure can be modified in various ways. For example, the learning support device 10 may be configured of a plurality of computers that are separated as hardware for the purpose of improving processing capability and reliability. For example, the functions of the receiving unit 60, the search unit 62, and the generation unit 63 and the function of the learning unit 64 are assigned to two computers in a distributed manner. In this case, the learning support device 10 is configured of the two computers.

As described above, the hardware configuration of the computer of the learning support device 10 can be changed as appropriate depending on required performance such as processing capacity, safety, and reliability. Further, it goes without saying that, in addition to the hardware, an application program such as the operation program 50 can be duplicated or distributed and stored in a plurality of storages for the purpose of securing the safety and the reliability.

In the above-described embodiment, for example, the following various processors can be used as a hardware structure of processing units that execute various types of processing, such as the receiving unit 60, the RW control unit 61, the search unit 62, the generation unit 63, the learning unit 64. As described above, the various processors include, in addition to the CPU 42 that is a general-purpose processor which executes software (operation program 50) to function as various processing units, a programmable logic device (PLD) that is a processor of which a circuit configuration can be changed after manufacture, such as a field programmable gate array (FPGA), and a dedicated electrical circuit that is a processor having a circuit configuration which is designed for exclusive use to execute specific processing, such as an application specific integrated circuit (ASIC).

One processing unit may be configured of one of the various processors or may be configured of a combination of two or more processors of the same type or different types (for example, a combination of a plurality of FPGAs and/or a combination of a CPU and an FPGA). In addition, a plurality of processing units may be configured of one processor.

As an example of configuring the plurality of processing units with one processor, first, there is a form in which, as typified by computers such as a client and a server, one processor is configured of a combination of one or more CPUs and software and the processor functions as the plurality of processing units. Second, there is a form in which, as typified by a system on chip (SoC) and the like, a processor that implements functions of an entire system including the plurality of processing units with one integrated circuit (IC) chip is used. As described above, various processing units are configured by using one or more of the various processors as a hardware structure.

In addition, more specifically, an electric circuit (circuitry) in which circuit elements, such as semiconductor elements, are combined can be used as the hardware structure of these various processors.

It is possible to understand the technologies described in following Appendices from the above description.

### [Appendix 1]

A learning support device comprising:
a processor,
in which the processor is configured to
   acquire an original image that is a source of a learning input image for a machine learning model used to ensure monoclonality of a cell seeded in a container, and
   generate, as the learning input image, an artificial image in which an optical virtual image caused by illumination light onto the container is depicted by performing image processing on the original image.

### [Appendix 2]

The learning support device according to Appendix 1,
in which the optical virtual image includes a mirror image that is generated by overlapping a part of one cell and a convergent image that is generated by the illumination light being condensed by a lens effect of the cell, and
the processor is configured to determine which of the mirror image and the convergent image is to be depicted, according to user's designation or the original image.

### [Appendix 3]

The learning support device according to Appendix 1 or 2,
in which the processor is configured to change a parameter that determines an aspect of the optical virtual image.

### [Appendix 4]

The learning support device according to Appendix 3,
in which the optical virtual image includes a mirror image that is generated by overlapping a part of one cell, and
the parameter is at least one of a direction of a symmetry axis, a distance of the mirror image from the cell, or a density of the mirror image.

### [Appendix 5]

The learning support device according to Appendix 3 or 4,
in which the optical virtual image includes a convergent image that is generated by the illumination light being condensed by a lens effect of the cell,
the processor is configured to
   acquire an image in which the convergent image is shown, as the original image, and
   set a plurality of reference points on the convergent image shown in the original image, and
the parameter is at least one of a movement direction of the reference point or a movement distance of the reference point.

### [Appendix 6]

The learning support device according to Appendix 5,
in which the processor is configured to set the reference point on a boundary between a portion where light is condensed and a portion where light is not condensed in the convergent image shown in the original image.

### [Appendix 7]

The learning support device according to Appendix 6,
in which the processor is configured to change an area ratio between the portion where the light is condensed and the portion where the light is not condensed by moving the reference point according to the parameter.

In the technology of the present disclosure, the above-described various embodiments and/or various modification examples may be combined with each other as appropriate. In addition, the present disclosure is not limited to the above-described embodiments, and various configurations can be adopted without departing from the gist of the present disclosure. Further, the technology of the present disclosure extends to a storage medium that non-transitorily stores a program in addition to the program.

The above descriptions and illustrations are detailed descriptions of portions related to the technology of the present disclosure and are merely examples of the technology of the present disclosure. For example, description related to the above configurations, functions, actions, and effects is description related to an example of configurations, functions, actions, and effects of the parts according to the technology of the present disclosure. Thus, it is needless to say that unnecessary portions may be deleted, new elements may be added, or replacement may be made to the content of the above description and the content of the drawings without departing from the gist of the technique of the present disclosure. Further, in order to avoid complications and facilitate understanding of the parts related to the technology of the present disclosure, descriptions of common general knowledge and the like that do not require special descriptions for enabling the implementation of the technology of the present disclosure are omitted, in the contents described and shown above.

In the present specification, the term "A and/or B" is synonymous with the term "at least one of A or B". That is, the term "A and/or B" means only A, only B, or a combination of A and B. In addition, in the present specification, the same approach as "A and/or B" is applied to a case in which three or more matters are represented by connecting the matters with "and/or".

All documents, patent applications, and technical standards mentioned in the present specification are incorporated herein by reference to the same extent as in a case in which each document, each patent application, and each technical standard are specifically and individually described by being incorporated by reference.

## Claims

1. A learning support device comprising:
a processor,
wherein the processor is configured to
acquire an original image that is a source of a learning input image for a machine learning model used to ensure monoclonality of a cell seeded in a container, and
generate, as the learning input image, an artificial image in which an optical virtual image caused by illumination light onto the container is depicted by performing image processing on the original image.

2. The learning support device according to claim 1,
wherein the optical virtual image includes a mirror image that is generated by overlapping a part of one cell and a convergent image that is generated by the illumination light being condensed by a lens effect of the cell, and
the processor is configured to determine which of the mirror image and the convergent image is to be depicted, according to user's designation or the original image.

3. The learning support device according to claim 1,
wherein the processor is configured to change a parameter that determines an aspect of the optical virtual image.

4. The learning support device according to claim 3,
wherein the optical virtual image includes a mirror image that is generated by overlapping a part of one cell, and
the parameter is at least one of a direction of a symmetry axis, a distance of the mirror image from the cell, or a density of the mirror image.

5. The learning support device according to claim 3,
wherein the optical virtual image includes a convergent image that is generated by the illumination light being condensed by a lens effect of the cell,
the processor is configured to
acquire an image in which the convergent image is shown, as the original image, and
set a plurality of reference points on the convergent image shown in the original image, and
the parameter is at least one of a movement direction of the reference point or a movement distance of the reference point.

6. The learning support device according to claim 5,
wherein the processor is configured to set the reference point on a boundary between a portion where light is condensed and a portion where light is not condensed in the convergent image shown in the original image.

7. The learning support device according to claim 6,
wherein the processor is configured to change an area ratio between the portion where the light is condensed and the portion where the light is not condensed by moving the reference point according to the parameter.

8. An operation method of a learning support device, the operation method comprising:
acquiring an original image that is a source of a learning input image for a machine learning model used to ensure monoclonality of a cell seeded in a container; and
generating, as the learning input image, an artificial image in which an optical virtual image caused by illumination light onto the container is depicted by performing image processing on the original image.

9. An operation program of a learning support device, the operation program causing a computer to execute a process comprising:
acquiring an original image that is a source of a learning input image for a machine learning model used to ensure monoclonality of a cell seeded in a container; and
generating, as the learning input image, an artificial image in which an optical virtual image caused by illumination light onto the container is depicted by performing image processing on the original image.
